Europäisches Patentamt

European Patent Office

Office européen des brevets

(11) Publication number: **0 034 190**
**B1**

(12) **EUROPEAN PATENT SPECIFICATION**

(45) Date of publication of patent specification: **22.06.83**

(51) Int. Cl.³: **A 61 K 7/06**

(21) Application number: **80100731.1**

(22) Date of filing: **14.02.80**

(54) Creme rinses with hair holding properties.

(43) Date of publication of application:
**26.08.81 Bulletin 81/34**

(45) Publication of the grant of the patent:
**22.06.83 Bulletin 83/25**

(84) Designated Contracting States:
**BE DE FR GB NL SE**

(56) References cited:
**DE - A - 1 949 606**
**FR - A - 2 117 844**
**US - A - 4 065 422**

**JAPANESE PATENT GAZETTE, vol. 3, nr. 59,
19—05—1979, page 142 (C46) Derwent
Publications London GB**

**The Chemistry and Manufacture of Cosmetics,
M.G. De Navarre, Vol. 1 (sec.ed.), 1962, p. 339;
I. Soc. Cosmetics Chemists, Vol. 17, p. 19
(1966)**

**The file contains technical information
submitted after the application was filed and not
included in this specification**

(73) Proprietor: **Helene Curtis Industries, Inc.**
**4401 West North Avenue**
**Chicago, III. 60639 (US)**

(72) Inventor: **Wagman, Julius**
**6142 North Harding Avenue**
**Chicago III. 60659 (US)**

(74) Representative: **Splanemann, Rainer, Dipl.-Ing.
et al,**
**Patentanwälte Dipl.-Ing. R. Splanemann, Dr. B.
Reitzner Tal 13**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# 0 034 190

## Creme rinses with hair holding properties

### DESCRIPTION

Background of the Invention

In the treatment and dressing of hair in a beauty salon, or at home, a creme rinse is conventionally applied to freshly shampooed and towel-dried hair to detangle the hair, reduce the static therein, and make it easier to comb. The creme rinse is left on the hair for a short time and is then rinsed from the hair.

The creme rinses available today are effective for the foregoing purposes, but leave the hair excessively soft and unable to hold a set.

Hair fixatives, such as polyvinylpyrrolidone have been added to creme rinses to provide body to the hair, but they have little effect because they are substantially rinsed out with the rinsing of the creme rinses.

JP—A—7 935 222 and DE—A—1 949 606 refer to hair rinse compositions containing cationic surfactants, such as quaternary ammonium salts, and anionic polymers, such as polyacrylic acid or collagen protein hydrolysates, which are dissolved in organic solvents, such as ethanol or propylene glycol. These compositions are disadvantageous in that the solvents also dissolve and remove the natural oils on hair. Furthermore, if wet hair is treated with these compositions, the solutions may become unstabilized, and insoluble products may be precipitated in an uneven manner when some portions of the hair have more water than other portions.

Summary of the Invention

In accordance with the present invention, a creme rinse composition is provided for application to the hair comprising an aqueous suspension containing from 0.02 to 2 weight percent of an anionic polymer and from 0.1 to 5 weight percent of a cationic surfactant capable of forming and having formed a water insoluble reaction product with said anionic polymer said reaction product being suspended in a liquid suspension medium in said composition, which liquid suspension medium consists essentially of water.

It has been found that the aforementioned insoluble reaction product is substantive to hair and remains on the hair to impart body and stiffness thereto, being detectable on the hair by a scanning electron microscope.

In a preferred aspect of this invention, the creme rinse composition contains a water-dispersible thickening agent to increase the viscosity of the composition and thereby keep the insoluble reaction product in suspension for prolonged periods. The preferred thickening agent is hydroxyethyl cellulose. Other suitable thickeners include gum arabic, gum karaya, ghatti gum, locust bean gum, guar gum, Irish moss, methyl cellulose, hydrolyzed starches and low molecular weight ethylene oxide polymers. The thickeners are suitable used in amounts from 0.1 to 5 percent by weight depending on the nature of the thickener and the viscosity desired. A preferred range is from 0.2 to 3 percent by weight.

Suitable cationic surfactants include quaternary ammonium chlorides and bromides having at least one long chain alkyl group or at least one aryl group. Specific surfactants which have been found suitable include oleyl dimethyl benzyl ammonium chloride, cetyl trimethyl ammonium chloride and methyl bis[2-hydroxyethyl] oleyl ammonium chloride. The cationic surfactant is generally used in amounts from 0.1 to 5 weight percent. A preferred range is from 0.2 to 3 weight percent by weight.

Suitable anionic polymers include vinyl polymers having a plurality of free carboxylic acid groups in the polymer chain, or alkali metal salts thereof. Specific anionic polymers which have been found to be useful include hydrolyzed copolymers of methyl vinyl ether and maleic anhydride, and the sodium salts of ethyl half esters of copolymers of methyl vinyl ether and maleic anhydride, the mol ratio of the methyl vinyl ether and maleic anhydride in these polymers ranging from 25:75 to 75:25. Hydrolyzed copolymers of ethylene oxide and maleic anhydride in mol ratios ranging from 75:25 to 25:75 are also useful. Other useful anionic polymers include the sodium salts of terpolymers of octyl acrylamide, an acrylate ester and butylaminoethyl methacrylate and acrylic acid polymers cross-linked with a polyfunctional agent such as a polyol or a polyamine. The anionic polymer is generally used in amounts from 0.02 to 2 weight percent, and preferably from 0.05 to 1 weight percent.

Sodium carboxymethyl cellulose may also be used as the anionic polymer in the compositions of this invention. Sodium carboxymethyl cellulose is known to be a thickening agent for aqueous systems. In combination with a cationic surfactant, however, it has little or no thickening effect unless it is used in substantial stoichiometric excess.

The relative proportions of anionic polymer and cationic surfactant are not critical. Satisfactory results are obtained with substantial stoichiometric excesses of either the anionic polymer or the cationic surfactant. It has also been found in compositions which have been centrifuged that the supernatant liquid is cationic even when the composition is made with a substantial stoichiometric excess of the anionic polymer.

The compositions may also contain coloring, perfumes and preservatives, if desired. Glutaraldehyde is a preferred preservative.

The compositions of this invention, when applied to freshly shampooed and towel-dried hair, and thereafter rinsed off, provide ease of detangling, ease of combing and static control and also provide body and set holding properties to the hair after drying. In addition, the compositions make the hair shaft surfaces resistant to the spread of sebaceous oils produced by the scalp and therefore tend to keep the hair cleaner for extended periods. The latter effect may be seen in photomicrographs which show a large contact angle between the hair and an oily material when a droplet of vegetable oil is applied to the hair.

### Example 1

A creme rinse formulation was prepared in accordance with the following formulation:

|  | Parts by Weight |
|---|---|
| Gantrez AN 119 (Note 1) | 0.29 |
| Ammonyx KP (Note 2) | 1.40 |
| Water | 98.31 |
|  | 100.00 |

*Note 1* — totally hydrolyzed water soluble 50:50 molar ratio copolymer of methyl vinyl ether and maleic anhydride with an average molecular weight of about 20,000.
*Note 2* — oleyl dimethyl benzyl ammonium chloride — 50% active.

### Example 2

A creme rinse formulation was prepared in accordance with the following formulation:

|  | Parts by Weight |
|---|---|
| Gantrez AN 119 | 0.18 |
| Ammonyx KP | 0.70 |
| Natrosol 250 HHR (Note 3) | 0.76 |
| Water | 98.36 |
|  | 100.00 |

*Note 3* — hydroxyethyl cellulose.

### Example 3

A creme rinse formulation was prepared in accordance with the following formulation:

|  | Parts by Weight |
|---|---|
| Gantrez AN 119 | 0.15 |
| Barquat CT 429 (Note 4) | 1.21 |
| Natrosol 250 HHR | 0.75 |
| Water | 97.89 |
|  | 100.00 |

*Note 4* — cetyl triethyl ammonium chloride — 29% active.

### Example 4

A creme rinse formulation was prepared in accordance with the following formulation:

|  | Parts by Weight |
|---|---|
| Gantrez AN 119 | 0.15 |
| Ethoquad 0/12 (Note 5) | 0.47 |
| Natrosol 250 HHR | 0.74 |
| Water | 98.64 |
|  | 100.00 |

*Note 5* — methyl bis[2-hydroxyethyl] oleyl ammonium chloride — 75% active.

3

0 034 190

## Example 5
A creme rinse formulation was prepared in accordance with the following formulation:

|  | Parts by Weight |
|---|---|
| Gantrez AN 119 | 0.200 |
| Ammonyx KP | 1.600 |
| Natrosol 250 HHR | 1.400 |
| Sodium Hydroxide | 0.034 |
| Glutaraldehyde | 0.050 |
| Water | 96.716 |
|  | 100.000 |

## Example 6
A creme rinse formulation was prepared in accordance with the following formulation:

|  | Parts by Weight |
|---|---|
| Gantrez AN 179 (Note 6) | 0.4 |
| Stearyl dimethyl benzyl ammonium chloride | 2.0 |
| Natrosol 250 HHR | 1.2 |
| Water | — to 100.0 parts |
| NaOH | to adjust pH to 3.5—4 |

*Note 6*— Totally hydrolyzed water soluble 50:50 molar copolymer of methyl vinyl ether and maleic anhydride with an average molecular weight of about 80,000.

## Example 7
A creme rinse formulation was prepared in accordance with the following formulation:

|  | Parts by Weight |
|---|---|
| Sodium carboxymethyl cellulose | 0.20 |
| Ammonyx KP | 1.60 |
| Natrosol 250 HHR | 1.40 |
| Water | 96.80 |
|  | 100.00 |

All of the above compositions provided ease of combability when applied to wet, towel-dried hair and provided hair holding properties when the hair was dried.

## Example 8
A creme rinse formulation was prepared in accordance with the following formulation:

|  | Parts by Weight |
|---|---|
| Gantrez ES 225 (Note 7) | 0.4 |
| Ammonyx KP | 1.6 |
| Natrosol 250 HHR | 1.4 |
| Water | 96.6 |
|  | 100.0 |

*Note 7* — sodium salt of the ethyl half ester of 50:50 molar copolymer of methyl vinyl ether and maleic acid

4

## Example 9
A creme rinse formulation was prepared in accordance with the following formulation:

|  | Parts by Weight |
|---|---|
| Carbomer 940 (Note 8) | 0.2 |
| Ammonyx KP | 1.6 |
| Natrosol 250 HHR | 1.4 |
| Glutaraldehyde | 0.05 |
| Water | 96.75 |
|  | 100.00 |

*Note 8* — polymer of acrylic acid cross-linked with a polyfunctional agent

## Example 10
A creme rinse formulation was prepared in accordance with the following formulation:

|  | Parts by Weight |
|---|---|
| Amphomer (Note 9) | 0.2 |
| Ammonyx KP | 0.8 |
| Natrosol 250 HHR | 1.4 |
| Water | 97.6 |
|  | 100.00 |

*Note 9* — sodium salt of octyl acrylamide/acrylates/butyl aminoethyl methacrylate polymer

All of the formulations of Examples 7 to 9 provided ease of combability when applied to wet, towel-dried hair and provided hair holding properties when the hair was dried.

## Examples 11 to 13
Creme rinse formulations were prepared in accordance with the following formulations:

|  | Parts by Weight | | |
|---|---|---|---|
|  | Ex. 11 | Ex. 12 | Ex. 13 |
| Natrosol 250 HHR | 1.4 | 1.4 | 1.4 |
| Gantrez AN 119 | 0.2 | 0.2 | 0.1 |
| Ammonyx KP | 1.6 | 0.8 | 1.6 |
| Water | 96.75 | 97.55 | 96.85 |
| Glutaraldehyde | 0.05 | 0.05 | 0.05 |
|  | 100.00 | 100.00 | 100.00 |

Mol Ratio — $\dfrac{\text{Equivalents AN 119}}{\text{Equivalents KP}}$

|  | | | |
|---|---|---|---|
|  | 0.7 | 1.4 | 2.8 |

The formulations of Examples 11, 12 and 13 all provided ease of wet combing, east of detangling, static control and stiffening of the hair. However, the formulation of Example 1 gave a greater stiffening effect than the formulation of Examples 12 or 13.

The formulations of Examples 11, 12 and 13 were made without thickener so that the solids therein could be readily separated by centrifugation. The formulations of Examples 11 and 12 were centrifuged for one hour to separate solids from supernatant liquids of milky appearance. The supernatant liquids were found to be cationic.

## Example 14
A creme rinse formulation was prepared in accordance with the following formulation:

|  | Parts by Weight |
|---|---|
| Methocel E4M (Note 10) | 1.4 |
| EMA—31 (Note 11) | 0.4 |
| Ammonyx KP | 3.2 |
| Water | 95.0 |
|  | 100.0 |

5

**0 034 190**

*Note 10* — methyl cellulose having a viscosity of 4000 cp at 2% concentration in $H_2O$ at 20°C.

*Note 11* — totally hydrolyzed water soluble 50:50 molar copolymer of ethylene oxide and maleic anhydride with an average molecular weight of about 100,000. The composition provided ease of combability when applied to wet, towel-dried hair and provided hair holding properties when the hair was dried.

**Claims**

1. A creme rinse composition for application to the hair comprising an aqueous suspension containing from 0.02 to 2 weight percent of an anionic polymer and from 0.1 to 5 weight percent of a cationic surfactant capable of forming and having formed a water insoluble reaction product with said anionic polymer, said reaction product being suspended in a liquid suspension medium in said composition, which liquid suspension medium consists essentially of water.

2. The creme rinse composition of claim 1 wherein said anionic polymer is a hydrolyzed copolymer of methyl vinyl ether and maleic anhydride in a mol ratio from 25:75 to 75:25.

3. The creme rinse composition of claim 1 wherein said anionic polymer is a sodium salt of an ethyl half ester of a copolymer of methyl vinyl ether and maleic anhydride in a mol ratio from 25:75 to 75:25.

4. The creme rinse composition of claim 1 wherein said anionic polymer is an acrylic acid polymer cross linked with a polyfunctional agent of the group consisting of polyols and polyamines.

5. The creme rinse composition of claim 1 wherein said cationic surfactant is oleyl dimethyl benzyl ammonium chloride.

6. The creme rinse composition of claim 1 wherein said cationic surfactant is cetyl trimethyl ammonium chloride.

7. The creme rinse composition of claim 1 wherein said cationic surfactant is methyl bis[2-hydroxy] oleyl ammonium chloride.

8. The creme rinse composition of claim 1 wherein said composition contains from 0.1 to 5 weight percent of a thickening agent.

9. The creme rinse composition of claim 9 wherein said thickening agent is hydroxyethyl cellulose.

10. The creme rinse composition of claim 1 wherein said anionic polymer is sodium carboxymethyl cellulose.

**Revendications**

1. Une composition de crème de rinçage pour application aux cheveux comprenant une suspension aqueuse contenant de 0,02 à 2 pour cent en poids d'un polymère anionique et de 0,1 à 5 pour cent en poids d'un agent tensio-actif cationique capable de former et ayant formé un produit de réaction insoluble dans l'eau avec le polymère anionique, ce produit de réaction étant en suspension dans un milieu liquide de suspension dans la composition, le milieu liquide de suspension étant constitué essentiellement d'eau.

2. Une composition de crème de rinçage selon la revendication 1, dans laquelle le polymère anionique est un copolymère hydrolysé d'oxyde de méthyle et de vinyle et d'anhydride maléique dans un rapport molaire compris centre 25:75 et 75:25.

3. Une composition de crème de rinçage selon la revendication 1, dans laquelle de polymère anionique est un sel de sodium d'un éthyl semi-ester d'un copolymère d'oxyde de méthyle et de vinyle et d'anhydride maléique dans un rapport molaire compris entre 25:75 et 75:25.

4. Une composition de crème de rinçage selon la revendication 1, dans laquelle le polymère anionique est un polymère d'acide acrylique réticulé avec un agent polyfonctionnel du groupe constitué par les polyols et les polyamines.

5. Une composition de crème de rinçage selon la revendication 1, dans laquelle l'agent tensio-actif cationique est le chlorure d'oléyl diméthyl benzyl ammonium.

6. Une composition de crème de rinçage selon la revendication 1, dans laquelle l'agent tensio-actif cationique est le chlorure de cétyl triméthyl ammonium.

7. Une composition de crème de rinçage selon la revendication 1, dans laquelle l'agent tensio-actif cationique est le chlorure de méthyl bis(2-hydroxy) oléyl ammonium.

8. Une composition de crème de rinçage selon la revendication 1, dans laquelle la composition contient de 0,1 à 5 pour cent en poids d'un agent épaississant.

9. Une composition de crème de rinçage selon la revendication 9, dans laquelle l'agent épaississant est l'hydroxyéthyl cellulose.

10. Une composition de crème de rinçage selon la revendication 1, dans laquelle le polymère anionique est la carboxyméthyl cellulose sodique.

6

# 0 034 190

**Patentansprüche**

1. Cremige Spülmasse für die Haarbehandlung in Form einer wäßrigen Suspension, die 0,02 bis 2 Gew.-% eines anionischen Polymers und 0,1 bis 5 Gew.-% eines kationischen Tensids enthält, das in der Lage ist, ein wasserunlösliches Reaktionsprodukt mit dem anionischen Polymer zu binden bzw. ein solches Produkt gebildet hat, wobei das Reaktionsprodukt in einem flüssigen Suspensionsmedium, das im wesentlichen aus Wasser besteht, in der genannten Masse suspendiert ist.

2. Cremige Spülmasse nach Anspruch 1, worin das anionische Polymer ein hydrolysiertes Copolymer von Methylvinyläther und Maleinsäureanhydrid in einem Molverhältnis von 25:75 bis 75:25 darstellt.

3. Cremige Spülmasse nach Anspruch 1, worin das anionische Polymer ein Natriumsalz eines Ethyl-Halbesters eines Copolymers von Methylvinyläther und Maleinsäureanhydrid im Molverhältnis 25:75 bis 75:25 darstellt.

4. Cremige Spülmasse nach Anspruch 1, worin das anionische Polymer ein Acrylsäurepolymer darstellt, das mit einem polyfunktionellen Mittel aus der Gruppe der Polyole und Polyamine vernetzt ist.

5. Cremige Spülmasse nach Anspruch 1, worin das kationische Tensid Oleyldimethylbenzylammoniumchlorid darstellt.

6. Cremige Spülmasse nach Anspruch 1, worin das kationische Tensid Cetyltrimethylammoniumchlorid darstellt.

7. Cremige Spülmasse nach Anspruch 1, worin das kationische Tensid Methyl-bis-[2-hydroxy]-oleylammoniumchlorid darstellt.

8. Cremige Spülmasse nach Anspruch 1, welche 0,1 bis 5 Gew.-% eines Verdickungsmittels enthält.

9. Cremige Spülmasse nach Anspruch 1, worin das Verdickungsmittel Hydroxyethylcellulose darstellt.

10. Cremige Spülmasse nach Anspruch 1, worin das anionische Polymer Natrium-Carboxymethylcellulose darstellt.